# FASCICULE DE BREVET EUROPEEN

(11) **EP 1 238 255 B1**
(45) Date de publication et mention de la délivrance du brevet: **03.05.2006**
(21) Numéro de dépôt: 00993441.5
(22) Date de dépôt: 12.12.2000
(51) Int. Cl.: G01N 1/08, G01N 33/12, A61B 10/00

(54) **DISPOSITIF ET PROCEDE DE PRELEVEMENT D'ECHANTILLON BIOLOGIQUE**
GERÄT UND VERFAHREN ZUR ENTNAHME EINER BIOLOGISCHEN PROBE
DEVICE AND METHOD FOR TAKING A BIOLOGICAL SAMPLE

(30) Priorité: 17.12.1999 FR 9916018
(43) Date de publication de la demande: 11.09.2002
(73) Titulaire: Bio-Rad Pasteur, 92430 Marnes-la-Coquette (FR)
(72) Inventeur: RASTORGOUEFF, Michel, F-78170 La Celle Saint Cloud (FR); DESLYS, Jean-Philippe, F-78150 Le Chesnay (FR); COMOY, Emmanuel, 91190 Saint Aubin (FR)
(74) Mandataire: Gillard, Marie-Louise
(86) Numéro de dépôt international: PCT/FR2000/003476
(87) Numéro de publication internationale: WO 2001/044782

(56) Documents cités:
- WO-A-97/36160
- WO-A-99/23950
- DE-U- 9 414 070
- US-A- 4 116 247
- US-A- 4 549 612
- US-A- 5 423 809
- US-A- 5 810 806
- US-A- 5 823 971

## Description

L'invention concerne un dispositif de prélèvement d'échantillon biologique mou, et un procédé le mettant en oeuvre.

On connaît depuis des décennies des essais d'analyse biologique qui mettent en oeuvre des réactifs ayant une affinité l'un pour l'autre. Ils mettent en jeu un échantillon biologique soupçonné contenir un analyte et un ou plusieurs réactifs présentant une affinité ou une aptitude à réagir avec l'analyte de l'échantillon.

L'échantillon est le plus souvent un fluide corporel de patient ou d'individu testé tel qu'un échantillon de sang total, de plasma, de sérum, d'urine, de liquide céphalo-rachidien, etc. L'échantillonnage ou prélèvement de fluide biologique peut se faire simplement, à l'aide de tout dispositif adapté tel qu'une pipette, une seringue, un automate de prélèvement... Le cas échéant, il peut requérir au préalable un filtrage pour éliminer toute sorte de débris ou microorganismes indésirables. D'une manière générale, les modes de prélèvement de fluides corporels réalisés quotidiennement sont satisfaisants : ils sont en général reproductibles, donnant des résultats quantitatifs et les dispositifs mis sur le marché sont fiables et relativement bon marché.

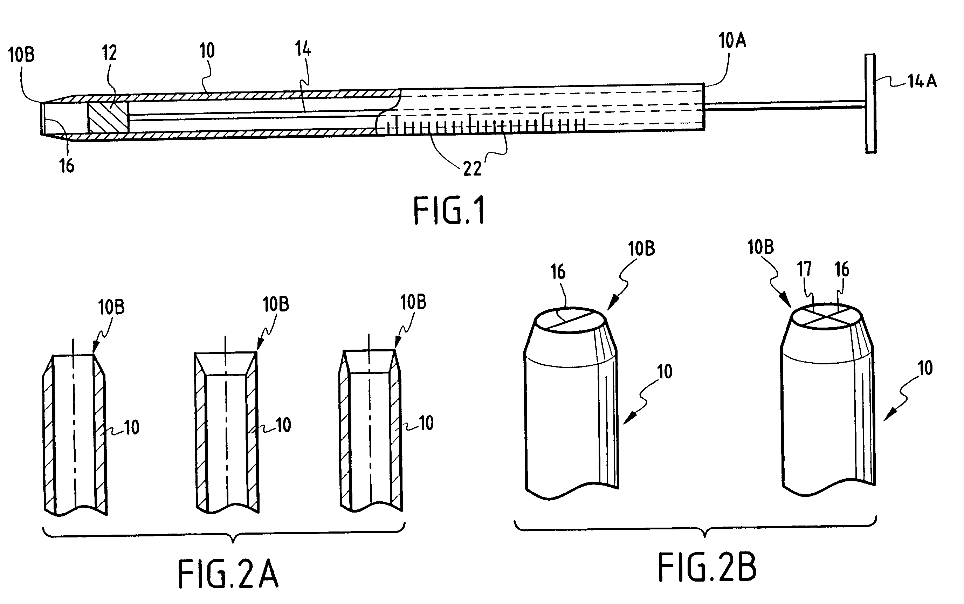

L'échantillon peut être aussi constitué d'un solide biologique tel qu'un fragment d'organe, ou de tissu, qui ne se répartit pas de façon systématiquement homogène dans le dispositif de prélèvement. Dans ce cas, le mode de prélèvement de l'échantillon étant beaucoup plus complexe, les dispositifs doivent souvent être appropriés à chaque cas, en fonction de la plus ou moins grande fluidité ou viscosité de ces solides corporels. A cet effet, on peut citer :
- Le brevet US 5 823 971 qui a pour objet un appareil pour réaliser des biopsies, tel qu'illustré par les Figures 2 à 7. Le dispositif selon les figures 2-2e comprend un tube interne 20 et un élément coupant externe 22. L'extrémité 24 du tube interne 20 est un bord de découpe. Une butée 23 limite le volume d'échantillon prélevé et permet sa restitution.

Cette butée peut seulement être déplacée vers l'extrémité libre du tube.

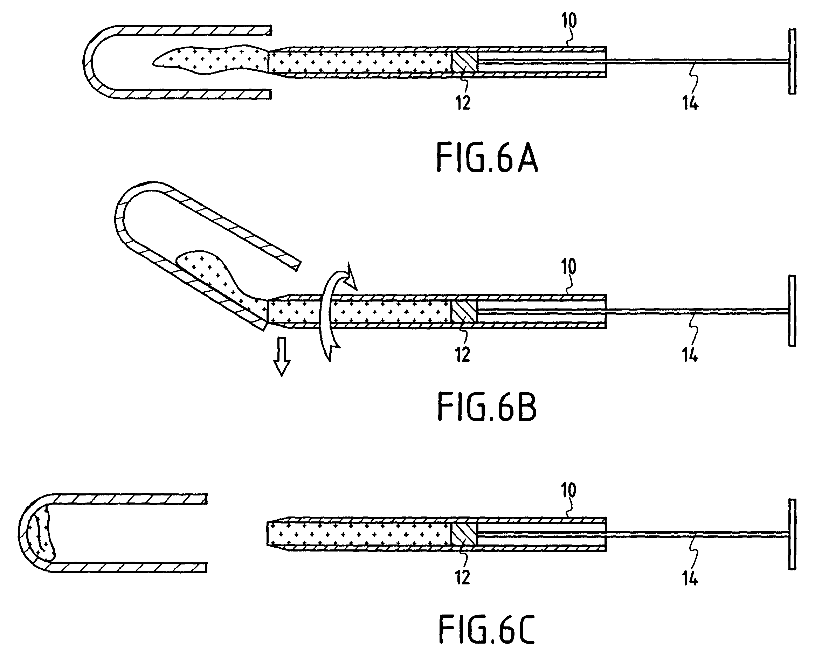

Le dispositif selon la figure 6 comporte un tube 100 ayant une extrémité distale 102 dont la forme permet la pénétration du dispositif dans le tissu. Il comporte également un fil de découpe 104 permettant d'obtenir deux parties égales d'un échantillon de tissu et une butée analogue à la butée 23 du dispositif selon les figures 2-2e.

Ces dispositifs doivent être couplés avec un tube flexible, type cathéter, permettant de les introduire au site du prélèvement.
- Le brevet US 5 810 806 qui décrit un dispositif de prélèvement d'échantillon biologique mou comprenant un tube dans lequel est inséré un piston pouvant être déplacé vers l'avant et vers l'arrière dans le tube et des fils de découpe placés à l'extrémité du tube.
- Le brevet US 4 549 612 qui concerne un dispositif de prélèvement de terre sans abîmer les racines.

Ce dispositif comprend un tube dans lequel coulisse un piston qui limite le volume de l'échantillon prélevé et permet sa restitution. Le dispositif est muni de moyens de repérage visibles pour repérer un volume correspondant à une variation de la position du piston dans le tube.

Les dispositifs décrits dans les brevets US 5 823 971 et US 5 810 806 ne présentent pas de moyens de repérage visibles pour repérer un volume correspondant à une variation de la position de la butée ou du piston dans le tube.
Le dispositif décrit dans le brevet US 4 549 612 ne comprend pas de fil de découpe disposé en travers de l'extrémité du tube.

Un très grand nombre d'analyses et de dépistages courants se font actuellement sur échantillons fluides. Tel n'est pas le cas des tests réalisés actuellement en diagnostic d'encéphalopathies spongiformes transmissibles (EST) qui sont des maladies neurologiques dégénératives telles que la " tremblante " (i.e. scrapie) du mouton, la " maladie de la vache folle ", encore appelée encéphalopathie spongiforme bovine (ci après " ESB ") chez les bovins, la maladie de Creutzfeldt-Jakob (MCJ) et le kourou (i .e. kuru) chez l'homme, et les encéphalopathies spongiformes transmissibles apparentées.

Dans le cas de l'ESB, il n'existe pas encore de diagnostic *in-vitro* courant faisable sur échantillon de fluide corporel, mais seulement sur prélèvements cérébraux de l'animal après abattage. L'examen post-mortem révèle des vacuolisations dans les cellules du tissu cérébral bovin et des dépôts d'un marqueur spécifique de cette maladie, la PrPres (forme anormale d'une protéine dénommée " protéine du prion "). Le diagnostic nécessite aujourd'hui que soit fait un prélèvement dans la matière cérébrale, notamment dans le tronc cérébral de l'animal, plus particulièrement au niveau des noyaux sensitifs et moteurs du nerf vague, qui constituent la zone d'accumulation préférentielle de la PrPres, marqueur diagnostique de l'ESB.

L'échantillon prélevé est alors soumis à divers traitements pour extraite la PrPres, marqueur spécifique de la maladie, qui est ensuite analysée par immunoessai.

L'incidence récente d'ESB en Grande Bretagne (depuis 1985), puis en Europe, est à l'origine d'un très gros problème de santé publique, vu la possible transmission de la maladie à l'homme, et son éradication est devenue par voie de conséquence un très grand enjeu économique.

Compte tenu de ses propriétés plastiques et de sa viscosité, la matière cérébrale bovine n'est pas facile à prélever de façon simple, rapide, reproductible, quantifiable et sûre. Or, il est essentiel que, pour un dépistage de masse sur carcasses bovines, les tests - et donc les prélèvements - soient réalisés de la façon la plus simple et utilisable dans un abattoir, la plus rapide après abattage, la plus reproductible, la plus quantifiable et la plus sûre, c'est-à-dire avec la meilleure sensibilité possible et sans contaminations extérieures.

Classiquement, lors du prélèvement d'un échantillon de matière cérébrale, en vue d'une analyse de la présence de PrPres, il est nécessaire d'utiliser un protocole lourd, impliquant de découper la tête, éventuellement d'ouvrir la boite crânienne, de saisir un morceau de matière cérébrale, par exemple à l'aide d'une cuillère, curette ou tout instrument approprié de ce genre, de découper manuellement au scalpel au moins une portion de ladite matière jusqu'à obtention du poids nécessaire qu'il faut ensuite vérifier à l'aide d'une balance. La lame de scalpel doit être changée à chaque prélèvement pour éviter toute contamination entre échantillons. Ce protocole s'avère donc en pratique peu commode et peu approprié au dépistage de masse.

L'opération de prélèvement doit, enfin, être aussi bon marché (matériel et main d'oeuvre) que possible, et ce, pour des raisons évidentes, pour le consommateur final.

Il existe donc un besoin urgent de disposer d'un dispositif de prélèvement d'échantillon biologique mou, en particulier de matière cérébrale, qui soit simple, qui soit utilisable, par exemple, dans un abattoir ou dans un laboratoire d'analyses, qui soit rapide de mise en oeuvre, économique, de performance reproductible, quantifiable, efficace et sauf de toute contamination extérieure. Il existe aussi un besoin urgent de disposer d'un procédé permettant de mettre en oeuvre ce type de prélèvement La présente invention s'est donné pour objet de pourvoir à ces besoins.

D'une manière générale, encore, il existe un tel besoin dans toutes les situations où il est nécessaire d'effectuer un prélèvement sur un échantillon biologique mou et pas seulement dans le seul domaine de la maladie spongiforme bovine ou de la tremblante du mouton.

Les inventeurs ont maintenant trouvé qu'il était possible d'obtenir un prélèvement de volume reproductible d'échantillon biologique mou à l'aide d'un dispositif équipé d'une extrémité tranchante, permettant d'obtenir une section nette de la matière molle constituant un échantillon. En particulier, les inventeurs ont découvert qu'il était possible de prélever une masse constante de matière molle à l'aide d'un dispositif cylindrique creux équipé en l'une de ses extrémités d'une section tranchante et porteuse d'un ou plusieurs fil(s) de découpe, positionné(s) diamétralement et perpendiculairement à l'axe dudit corps cylindrique creux, lorsque ledit dispositif cylindrique faisait l'objet d'une rotation suffisante sur lui-même.

Par "échantillon biologique mou", il faut comprendre un échantillon provenant d'une matière biologique de consistance telle qu'elle puisse être sectionnée sans effort par un outil tel qu'un scalpel. Comme cela a été indiqué précédemment, il s'agit par exemple de matière cérébrale.

Par " matière cérébrale ", il faut comprendre toute portion de la masse constituant le système nerveux central, et en particulier, mais non exclusivement, la partie anatomique classiquement dénommée " tronc cérébral " notamment centré sur les noyaux sensitifs et moteurs du nerf vague, qu'elle soit à l'état naturel ou qu'elle ait été traitée et, par exemple, obtenue sous forme de broyat pâteux.

L'invention a donc pour objet un dispositif de prélèvement d'échantillon biologique mou, notamment de matière cérébrale, comprenant un corps cylindrique creux, muni de deux ouvertures, une à chaque extrémité, et dans lequel, par une première extrémité, est inséré un piston muni d'une tige, ledit ensemble piston plus tige pouvant être déplacé vers l'avant et vers l'arrière dans ledit corps cylindrique creux, l'ouverture de la deuxième extrémité du corps cylindrique creux présentant un bord tranchant, ladite deuxième extrémité portant au moins un fil de découpe qui est disposé en travers de cette ouverture et est orienté perpendiculairement à l'axe dudit corps et ledit dispositif présentant des moyens de repérage visibles pour repérer un volume correspondant à une variation de la position du piston dans le corps cylindrique creux.

Le bord de la deuxième extrémité du corps cylindrique creux est suffisamment tranchant pour s'enfoncer dans l'échantillon biologique mou en le sectionnant. On pourra choisir un bord plus ou moins coupant selon la nature de l'échantillon.

Le corps cylindrique creux est avantageusement transparent et le piston est avantageusement opaque et coloré. Le corps cylindrique creux et le piston sont nécessairement choisis de sorte qu'ils permettent et assurent une étanchéité substantielle entre eux.

Le dispositif comporte avantageusement des moyens pour repérer un volume correspondant à une variation de la position du piston dans le corps.

Ces moyens comprennent, par exemple, un ou plusieurs repère(s) visible(s) situé(s) en une ou plusieurs position(s) distincte(s) sur le corps cylindrique creux et délimitant un ou plusieurs volume(s) cylindrique(s) défini(s). De préférence, mais non exclusivement, les repères visibles selon l'invention sont au nombre d'au moins deux.

Les " repères visibles ", encore appelés moyens de repérage, selon l'invention peuvent être constitués, par exemple, par une graduation classique comme celle représentée en Figure 1, ou par des dessins géométriques variés tels que, par exemple, des carrés, des cercles, des triangles, ou tout signe de ce genre, etc. disposés selon un " pas " permettant de sélectionner le volume désiré. Par le mot " pas ", on entend ici la distance existant entre deux repères identiques, par exemple et non exclusivement, la distance existant entre deux dessins géométriques identiques, deux triangles, deux carrés ou deux cercles.

En variante, les au moins deux repères visibles peuvent être situés sur la tige en au moins deux positions distinctes pour définir un volume cylindrique donné, lorsque la tige est déplacée d'un pas, c'est-à-dire d'une première position, dans laquelle le premier repère coïncide avec une zone donnée du corps cylindrique creux, vers une deuxième position, dans laquelle le deuxième repère coïncide avec ladite zone du corps cylindrique creux.

Avantageusement, le bord tranchant de la deuxième extrémité du corps cylindrique creux est formé par une réduction progressive de l'épaisseur de la paroi de ce corps donnant à sa terminaison une forme tronconique ou par une terminaison en biseau dudit corps.

Le fil de découpe est disposé diamétralement en travers de l'ouverture de la deuxième extrémité du corps cylindrique creux et est orienté perpendiculairement à l'axe dudit corps.

Pour réaliser un prélèvement d'échantillon biologique mou, à l'aide d'un dispositif selon l'invention, on procède selon les étapes suivantes :
(1) ledit dispositif est appliqué à la surface dudit échantillon, la deuxième extrémité du corps cylindrique creux étant en contact direct avec ledit échantillon, et l'ensemble piston plus tige étant alors poussé à fond vers l'avant, c'est-à-dire vers ladite deuxième extrémité,
(2) ledit corps cylindrique creux est enfoncé jusqu'au niveau désiré dans ledit échantillon, alors que le piston est maintenu au niveau de la surface dudit échantillon,
(3) lorsque le niveau désiré d'enfoncement dudit corps cylindrique creux est atteint, ledit corps cylindrique creux subit une rotation sur son axe pour découper l'échantillon à l'aide d'au moins un fil de découpe,
(4) l'ensemble constitué dudit corps cylindrique creux et dudit ensemble piston plus tige, maintenu tel quel, est retiré de l'échantillon.

Par la suite, l'échantillon prélevé pourra être extrudé du corps du dispositif, sectionné et libéré pour être distribué dans un récipient approprié en vue de la détection et/ou quantification de l'analyte contenu dans l'échantillon. Les opérations d'extrusion et de libération de l'échantillon seront décrites en détail ci-après.

Avantageusement, la rotation servant à la découpe de l'échantillon à l'aide du fil est opérée sur 360 degrés.

Les moyens de repérage précédemment évoqués sont mis à profit pour repérer un volume de prélèvement correspondant à un enfoncement donné du corps cylindrique creux dans l'échantillon et/ou pour repérer un volume d'analyse extrudé du corps du dispositif en vue d'être analysé.

Ainsi, lorsque ces moyens de repérage comprennent deux repères visibles, disposés sur le corps cylindrique creux, on peut enfoncer le corps cylindrique creux dans l'échantillon jusqu'à une position correspondant à un volume de prélèvement au moins égal au volume donné par le repère le plus éloigné de la deuxième extrémité du corps cylindrique creux. Ensuite, on peut conserver comme volume d'analyse une quantité donnée par le déplacement du piston vers la deuxième extrémité du corps cylindrique creux entre les deux repères.

Lorsque les deux repères visibles sont disposés sur la tige du piston, on peut enfoncer le corps cylindrique creux dans l'échantillon jusqu'à une position correspondant à un volume de prélèvement au moins égal au volume donné par le repère le plus proche de l'extrémité de la tige qui comporte le piston. Ensuite, on peut conserver comme volume d'analyse une quantité donnée par le déplacement du piston vers la deuxième extrémité du corps cylindrique creux entre deux positions, dans lesquelles ce repère puis l'autre repère coïncident successivement avec une zone donnée du corps cylindrique creux.

L'invention sera mieux comprise à l'aide des figures qui suivent et qui décrivent plus complètement le dispositif et le procédé selon l'invention à titre d'exemples non limitatifs.
La figure 1 montre un exemple, pour partie, en section longitudinale et pour partie en vue extérieure, du dispositif selon l'invention, porteur d'un exemple de repères visibles gradués.
Les figures 2A et 2B montrent plusieurs variantes de détails de réalisation de l'extrémité tranchante selon l'invention.
Les figures 3A à 3E illustrent la mise en oeuvre du procédé de prélèvement et d'extraction de l'échantillon conforme à l'invention.
Les figures 4A et 4B montrent un cas d'utilisation du dispositif pour réaliser l'extrusion, selon un pas défini par la distance entre les points C et D, de l'échantillon désiré après prélèvement.
Les figures 5A et 5B montrent une variante des figures 4A et 4B mettant en oeuvre une extrusion de l'échantillon désiré selon un pas défini par la distance entre les points A et B.
Les figures 6A, 6B et 6C montrent l'opération de " libération " de l'échantillon vers le récipient par rotation à 180 degrés d'un dispositif selon l'invention et râclement de l'extrémité tranchante 10B contre le bord supérieur du récipient.

Sur la figure 1, on voit que le dispositif représenté comporte un corps cylindrique creux transparent (10), à l'intérieur duquel est monté mobile un piston (12) commandé par une tige (14) dont l'extrémité libre opposée au piston est pourvue d'une tête d'actionnement (14A). La tige du piston émerge du tube par la première extrémité (10A) de ce dernier. La deuxième extrémité (10B) du tube présente un bord tranchant.

La paroi du tube présente une épaisseur sensiblement constante sur toute sa longueur, sauf dans la zone voisine de sa deuxième extrémité (10B). En effet, comme on le voit sur la figure 2A, celle-ci peut être rendue tranchante par une diminution progressive de l'épaisseur de la paroi du tube. Cette réduction d'épaisseur peut lui conférer une forme tronconique sur sa périphérie externe seulement comme le montre la partie gauche de la figure 2A. Elle peut lui conférer une forme tronconique sur sa périphérie interne seulement comme le montre la partie centrale de la figure 2A ou former deux surfaces tronconiques, respectivement sur la périphérie interne et sur la périphérie externe, comme le montre la partie droite de la figure 2A.

Un fil de découpe est disposé en travers de l'extrémité (10B) du tube. Comme le montre la partie gauche de la figure 2B, il peut s'agir d'un fil unique (16) disposé diamétralement dans le plan de l'extrémité libre de la partie d'extrémité (10B). Comme le montre la partie droite de la figure 2B, on peut en variante utiliser deux fils (16) et (17) disposés en croix dans le même plan.

Le fil peut être à section circulaire, triangulaire ou autre. De manière générale, toute section propre à lui donner un effet coupant, lorsqu'il est déplacé dans la matière molle de prélèvement, convient.

Le piston (12) peut être constitué par un élément rapporté monté à l'extrémité libre de la tige (14) opposée à sa tête d'actionnement (14A). Il peut également être formé monobloc avec cette tige.

### A) Prélèvement de l'échantillon :

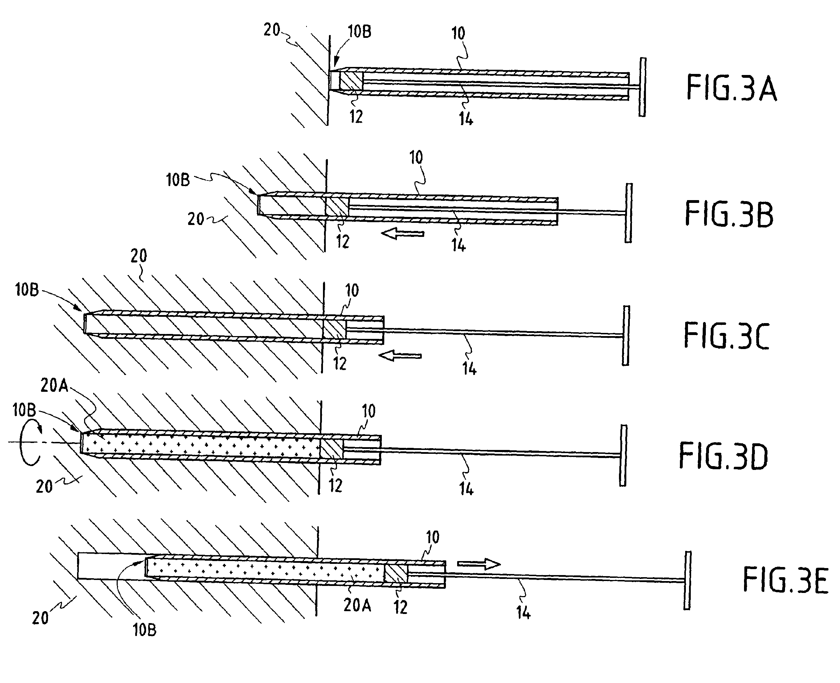

La figure 3A montre la première étape du protocole ou mise en oeuvre du procédé de prélèvement d'échantillon selon l'invention. Dans cette première étape, le dispositif est disposé de telle sorte que la deuxième extrémité (10B) du tube soit en contact avec l'échantillon mou (20) à prélever. A ce moment, le piston (12) est dans sa position la plus proche de cette deuxième extrémité.

La figure 3B montre l'étape suivante dite de carottage, c'est-à-dire l'enfoncement progressif et rectiligne du corps cylindrique creux (10) du dispositif dans l'échantillon mou, avec maintien simultané du piston au niveau de la surface de l'échantillon mou.

La figure 3C montre la fin du carottage, le corps cylindrique creux (10) ayant été enfoncé jusqu'à la position désirée.

La figure 3D illustre la découpe d'une portion de l'échantillon mou par rotation du corps cylindrique creux (10) du dispositif autour de son axe longitudinal. La rotation est réalisée selon une amplitude angulaire suffisante pour que le fil de découpe disposé à l'extrémité (10B) sépare totalement la partie (20A) d'échantillon contenue dans le corps cylindrique deux (10) du reste de l'échantillon mou (20). Par sécurité, on choisira avantageusement une amplitude angulaire de rotation de 360 degrés, voire davantage.

La figure 3B montre le prélèvement et l'extraction à partir de l'échantillon mou d'une portion de celui-ci. Cette étape consiste en le retrait de l'ensemble tube cylindrique deux et piston + tige, maintenu tel quel.

Par " maintenu tel quel " on entend le fait que, lors du retrait du dispositif selon l'invention, de l'échantillon, les positions relatives du corps cylindrique creux ou tube et de l'ensemble piston + tige sont maintenues inchangées.

Cette étape a pour effet d'extraire la " carotte" ou portion de l'échantillon recherché.

La portion d'échantillon effectivement prélevée est ensuite extrudée par refoulement pour être déposée ou distribuée dans un récipient convenable en vue de l'analyse à réaliser. L'étape d'extrusion + libération sera décrite ci-après et illustrée par les figures 6A, 6B et 6C.

D'une manière générale, dans le cadre de la détection d'un analyte, tel que par exemple une molécule de PrPres, cette portion extrudée et libérée d'échantillon est *in fine* récupérée dans un récipient, en soi connu de l'homme du métier et approprié, en vue de l'extraction éventuelle de l'analyte, comme c'est le cas le plus souvent pour une molécule de PrPres.

Une caractéristique essentielle de tout dispositif selon l'invention est constituée par la présence, sur une extrémité tranchante du corps cylindrique creux, d'au moins un fil de découpe, positionné diamétralement et perpendiculairement à la section dudit corps cylindrique creux.

Une caractéristique essentielle de tout procédé de prélèvement d'échantillon biologique mou selon l'invention est constituée par la rotation qui est imprimée au corps cylindrique creux en figure 3D du protocole d'utilisation du dispositif selon l'invention. C'est en effet une rotation suffisante, au minimum de 180 degrés, imprimée, lorsqu'une position - correspondant à un repère visible on non - donnée a été atteinte, au corps cylindrique creux, et donc au(x) fil(s) de découpe, qui permet la séparation nette et reproductible d'une fraction constante de l'échantillon, quelle que soit la consistance du matériau biologique mou constitutif de l'échantillon. L'effet de ladite rotation transforme, en effet, le(s) fil(s) de découpe en moyens tranchants.

### B) Extrusion de l'échantillon prélevé :

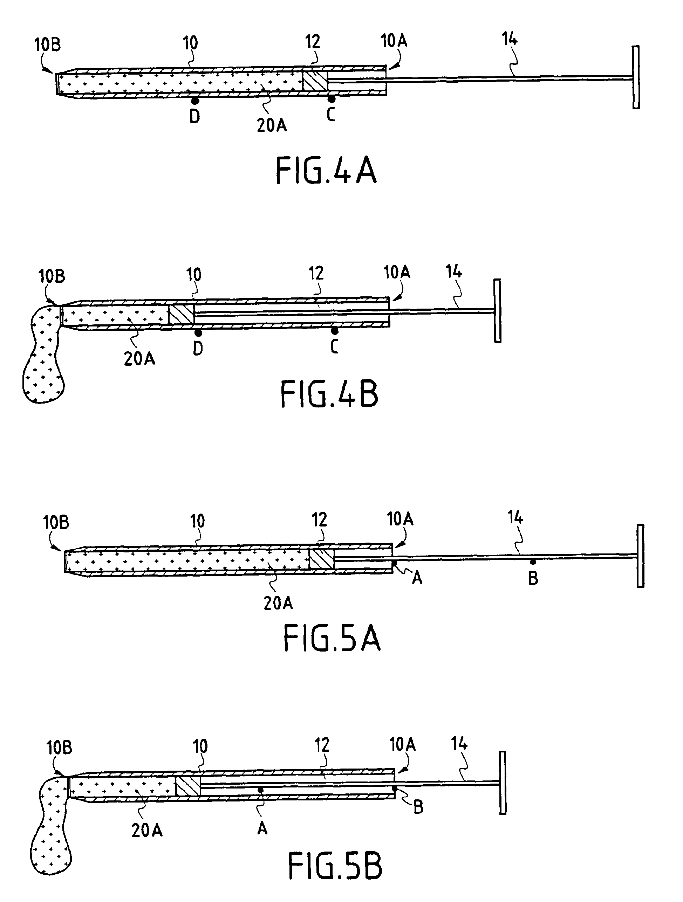

Dans un mode de réalisation particulièrement avantageux illustré par les figures 4A et 4B, le dispositif selon l'invention comprend un corps cylindrique creux (10) transparent pourvu de deux repères C et D visibles, situés entre ses deux extrémités (10A) et (10B), en deux positions distinctes désirées et délimitant un volume cylindrique défini, qui lui même permet de définir une masse définie d'échantillon mou à prélever. Cette caractéristique permet de repérer et suivre la progression de l'ensemble piston plus tige lors de l'extrusion hors du corps cylindrique creux transparent de l'échantillon prélevé. Pour réaliser (voir figure 4A) le prélèvement du volume (20A), défini par les repères C et D, le piston (12) a été déplacé vers l'extrémité (10A) au moins jusqu'au repère C le plus éloigné de l'extrémité (10B). Pour réaliser (voir figure 4B) l'extrusion hors du corps cylindrique creux transparent de l'échantillon prélevé, il suffit de repousser en sens inverse le piston (12) jusqu'au repère D. On pourra ensuite réaliser des analyses sur la quantité précise du prélèvement délimitée par un déplacement du piston d'un" pas " du repère C vers le repère D.

Dans un autre mode de réalisation particulièrement avantageux, le dispositif selon l'invention comprend un ensemble piston plus tige, dont la tige est pourvue de deux repères visibles A et B, situés entre ses deux extrémités, en deux positions distinctes désirées et définissant un volume cylindrique donné. Cette caractéristique permet de repérer et suivre la progression de l'ensemble piston plus tige lors de l'extrusion hors du corps cylindrique creux de l'échantillon prélevé. Pour réaliser (voir figure 5A) le prélèvement du volume (20A), défini par les repères A et B, le piston (12) a été déplacé vers l'extrémité (10A) au moins jusqu'à ce que le repère A, qui est le plus proche du piston (12), vienne en regard d'une zone déterminée du corps cylindrique creux transparent (10) qui, en l'espèce, est sa première extrémité (10A).

Pour réaliser (voir figure 5B) l'extrusion hors du corps cylindrique creux transparent (10) de l'échantillon prélevé, il suffit de repousser en sens inverse le piston (12) jusqu'à ce que le repère B vienne en regard de l'extrémité (10A) du corps cylindrique creux transparent (10). On pourra ensuite réaliser des analyses sur la quantité extrudée par le déplacement du piston entre la position de la figure 5A et celle de la figure 5B.

Dans un autre mode de réalisation particulièrement avantageux, le dispositif selon l'invention comprend un corps cylindrique creux transparent (10) gradué, c'est-à-dire portant une pluralité de graduations, sur toute sa longueur. Les graduations permettent de mesurer la profondeur atteinte, dans l'échantillon mou, par l'extrémité tranchante du dispositif de façon à effectuer la découpe de la carotte exactement au niveau souhaité. Ces graduations sont indiquées à titre d'exemple sur la figure 1, sur laquelle elles sont désignées par la référence (22). Les graduations permettent aussi de repérer le déplacement du piston pour mesurer le volume prélevé (figures 3A à 3D) aussi bien que pour mesurer la quantité extrudée (de façon similaire à celle représentée en figures 4A et 4B) qui servira aux analyses.

### C) Libération de l'échantillon prélevé :

Quel que soit le mode de mise en oeuvre du procédé de " prélèvement + extrusion " ci-dessus utilisé, à la fin du mouvement d'avancée (représenté en figure 4B ou 5B) du piston permettant de réaliser l'extrusion mesurée, voulue, d'échantillon, il faut séparer le morceau d'échantillon déjà sorti (i.e. extrudé) du corps cylindrique creux (10) du dispositif de celui restant dans ledit corps cylindrique creux. Ceci peut se faire de toute manière appropriée. De façon avantageuse, on pourra, pour ce faire, (voir les figures 6A, 6B et 6C) imprimer au dispositif un mouvement de rotation d'environ 180 degrés tout en faisant râcler l'extrémité tranchante (10B) du corps cylindrique creux contre le bord supérieur du récipient (qui peut être ici, par exemple, un tube à essai, un flacon, etc.) dans lequel on désire libérer finalement l'échantillon, le récipient étant avantageusement incliné de 45 degrés par rapport à l'axe longitudinal du dispositif pour faciliter la libération de l'échantillon. Ceci a pour effet de sectionner l'échantillon en deux et de libérer le morceau extrudé (c'est l'étape dite de " libération " de l'échantillon). Le mouvement de rotation d'environ 180 degrés ci-dessus permet en effet de profiter encore une fois de l'effet tranchant du fil pour améliorer la qualité et la reproductibilité de la découpe, donc de la libération de l'échantillon.

Les repères visibles et les graduations, évoqués ci-dessus, constituent un élément indispensable pour rendre reproductibles les quantités d'échantillon prélevées, soit dans un même échantillon, soit entre divers échantillons, à un niveau de profondeur précis et déterminable à l'avance par l'opérateur, ainsi que les quantités d'échantillon libérées.

Le corps cylindrique creux du dispositif selon l'invention peut être fabriqué en tout matériau solide et imperméable approprié, tel qu'un polymère comme le polypropylène ou tout autre matériau possédant substantiellement les mêmes caractéristiques physicochimiques, en particulier une neutralité chimique vis-à-vis de l'échantillon. Le corps cylindrique creux transparent du dispositif préféré est fabriqué dans du polypropylène.

Le piston du dispositif selon l'invention peut être fabriqué en tout matériau solide et imperméable approprié, tel qu'un polymère organique comme le silicone, le caoutchouc, synthétique ou naturel, le polyéthylène, le polypropylène, ou encore tout autre matériau possédant substantiellement les mêmes caractéristiques physicochimiques, c'est-à-dire une souplesse, en vue de l'étanchéité, et une neutralité chimique vis-à-vis de l'échantillon. Le piston du dispositif préféré est fabriqué en polyéthylène.

La tige du piston du dispositif selon l'invention peut être fabriquée en tout matériau solide approprié, tel qu'un polymère organique comme le polyéthylène, le polypropylène, ou tout autre matériau possédant substantiellement les mêmes caractéristiques physicochimiques. La tige du piston du dispositif préféré est fabriquée en polyéthylène ou en polypropylène.

L'extrémité tranchante et porteuse d'au moins un fil de découpe, le fil de découpe et le corps cylindrique creux ou tube transparent sont fabriqués, de préférence en même temps, par moulage par injection du polymère transparent choisi. L'empreinte monobloc dans le moule permet d'obtenir un dispositif fonctionnel à un coût de revient avantageux.

### Exemple 1

### Fabrication de dispositifs selon l'invention

Par moulage conventionnel par injection, en soi connu de l'homme du métier des matières plastiques, on a fabriqué en polypropylène plusieurs séries de dispositifs selon l'invention et correspondant exactement à la Figure 1, sauf que les graduations faites sur le corps cylindrique creux étaient remplacées par des signes visibles de type triangles, carrés et traits perpendiculaires permettant de définir, par déplacement, des volumes précis, selon la manière représentée en Figures 4A et 4B. Le diamètre intérieur du corps cylindrique creux était d'environ 0,5 centimètre et sa longueur d'environ 7,5 centimètres. La distance entre deux signes identiques (triangles, carrés ou traits perpendiculaires) marqués sur le corps cylindrique creux était d'environ 19,5 millimètres. Cette distance ou " pas " d'environ 19,5 millimettes a été choisie afin d'assurer, après prélèvement une éjection et une libération de 350 milligrammes + ou - 15% d'échantillon de tronc cérébral bovin.
Un fil unique de découpe était disposé en travers de l'extrémité (10B) desdits dispositifs comme cela est exactement représenté sur la figure 2B, schéma de gauche.

### Exemple 2

### Prélèvement de tronc cérébral bovin

Il a été réalisé plusieurs séries d'essai montrant que le dispositif selon l'invention obtenu dans l'exemple 1 (ci-après désigné " seringue ") permet de réaliser des prélèvements de tronc cérébral bovin de façon très reproductible.

On a tout d'abord prélevé le tronc cérébral de différents bovins que l'on a conservé dans différents sachets plastiques.

Le protocole de " prélèvement + extrusion + libération " d'échantillon alors suivi a été celui qui est décrit plus haut et schématisé sur les Figures 3A à 3E, les Figures 4A et 4B, suivi de la libération (Figures 6A à 6C) et de la pesée de l'échantillon libéré. Lors de l'extrusion de l'échantillon hors de la seringue, on faisait avancer le piston de la seringue d'un " pas ", par exemple, d'un triangle au triangle suivant (ou d'un carré au carré suivant,..), espacés l'un de l'autre de 19,5 millimètres, et selon la manière indiquée sur les Figures 4A et 4B. La portion de tronc cérébral extrudée hors de la seringue était ensuite sectionnée et libérée par rotation de 180 degrés du dispositif et râclement simultanés de l'échantillon sur le bord du tube récepteur selon le protocole décrit plus haut (voir figures 6A, 6B et 6C). La pesée de l'échantillon était enfin réalisée sur une balance de précision, sensible au 1/10^{ème} de milligramme.

On a ainsi réalisé deux séries d'essais à l'aide de deux lots différents de seringues.

### Série 1 (résultats regroupés dans le Tableau I) :

Un expérimentateur unique a donc ainsi effectué l'opération "prélèvement + extrusion + libération + pesée " d'échantillon de tronc cérébral à l'aide de 7 seringues différentes d'un même premier lot. Pour cette étude, il a répété cette opération plusieurs fois avec chaque seringue. Le nombre total d'essais réalisés, toutes seringues confondues, a été de 49.

Le Tableau I ci-après récapitule les résultats obtenus.

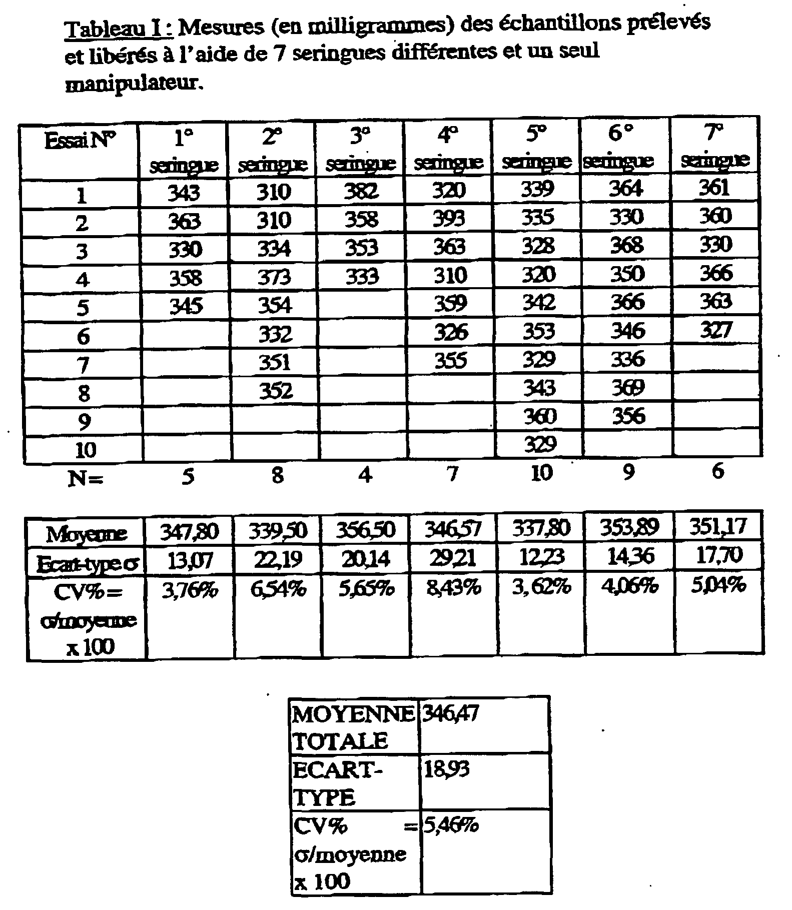

Les coefficients de variation calculés (désignés par "CV%") dans le Tableau I reflètent la reproductibilité de l'opération "prélèvement + extrusion + libération + pesée " d'échantillon de tronc cérébral à l'aide de la seringue selon l'invention.

Les inventeurs ont constaté la remarquable reproductibilité globale interseringues (CV% = 5,46% pour n = 49 essais) des résultats obtenus avec la seringue selon l'invention. Une telle reproductibilité est a priori surprenante pour l'homme du métier, compte tenu de la nature de la matière première, d'une part, et de la simplicité (rusticité) du dispositif utilisé, d'autre part.

### Série 2 (résultats regroupés sur le Tableau II) :

Trois manipulateurs ont réalisé cinq essais par seringue sur un nombre variable de seringues différente provenant d'un deuxième lot fabriqué selon le mode opétatoire de l'exemple 1.

**Tableau II : mesures (en milligrammes) des échantillons prélevés et libérés à l'aide de diverses seringues et réalisées par 3 manipulateurs.**

| Manipulateur A | | | | | | |
|---|---|---|---|---|---|---|
| Essai N° | 1° seringue | 2° seringue | 3° seringue | 4° seringue | 5° seringue | |
| 1 | 364 | 334 | 387 | 375 | 320 | |
| 2 | 337 | 361 | 344 | 352 | 323 | |
| 3 | 350 | 359 | 358 | 354 | 387 | |
| 4 | 375 | 300 | 370 | 350 | 364 | |
| 5 | 372 | 349 | 353 | 345 | 376 | TOTAL |
| Moyenne | 359,6 | 340,6 | 362,4 | 355,2 | 354,0 | 354 |
| Ecart-type σ | 15,9 | 25,1 | 16,7 | 11,6 | 30,8 | 20,8 |
| CV% = σ/moyenne x 100 | 4,42% | 7,37% | 4,61% | 3,27% | 8,70% | 5,88% |
| | | | | | | |

| Manipulateur B | | | | | | |
|---|---|---|---|---|---|---|
| Essai N° | 1° seringue | 2° seringue | 3° seringue | | | |
| 1 | 337 | 333 | 392 | | | |
| 2 | 382 | 322 | 396 | | | |
| 3 | 332 | 372 | 382 | | | |
| 4 | 362 | 368 | 364 | | | |
| 5 | 344 | 380 | 376 | | | TOTAL |
| Moyenne | 351,4 | 355,0 | 382,0 | | | 363 |
| Ecart-type σ | 20,5 | 25,8 | 12,8 | | | 23,6 |
| CV% = σ/moyenne x 100 | 5,83% | 7,27% | 3,35% | | | 6,50% |

| Manipulateur C | | | | | | |
|---|---|---|---|---|---|---|
| Essai N° | 1° seringue | 2° seringue | 3° seringue | | | |
| 1 | 342 | 357 | 393 | | | |
| 2 | 365 | 402 | 355 | | | |
| 3 | 347 | 338 | 315 | | | |
| 4 | 310 | 411 | 343 | | | |
| 5 | 369 | 381 | 329 | | | TOTAL |
| Moyenne | 346.6 | 377.8 | 347,0 | | | 357 |
| Ecart-type σ | 23,5 | 30,5 | 29,8 | | | 30,1 |
| CV% = σ/moyenne x 100 | 6,78% | 8,07% | 8,59% | | | 8,43% |

Reproductibilité globale entre les essais des manipulateurs A, B et C

| | |
|---|---|
| MOYENNE TOTALE | 357 |
| ECART-TYPE | 24,2 |
| CV% = σ/moyenne x 100 | 6,78% |

Les inventeurs ont à nouveau constaté la remarquable reproductibilité globale inter-seringues et inter-manipulateurs (CV% = 6,78% pour n = 55 essais) des résultats obtenus avec la seringue selon l'invention. Cette reproductibilité montre donc bien que la seringue selon l'invention est facile à manipuler et d'un apprentissage aisé pour tout homme du métier.

Il est donc désormais aisé et justifié, avec la seringue selon l'invention, d'éviter de peser les échantillons à analyser, en vue de déterminer si un échantillon de tronc cérébral ou d'une autre matière cérébrale est porteur de PrPres ou de tout autre marqueur d'encéphalopathie spongiforme de tout type: bovin et ovin, notamment. Le dispositif selon l'invention, en particulier la seringue, est d'application diagnostique générale à toutes ces pathologies.

La durée totale de l'opération " prélèvement + extrusion + libération " de l'échantillon est d'environ une minute pour un manipulateur, après quelque entraînement. C'est donc une opération particulièrement rapide.

En outre, l'opération " prélèvement + extrusion + libération" de l'échantillon, grâce aux seringues selon l'invention et aux sacs plastiques utilisés, a évité de souiller les gants et l'environnement du manipulateur. Cette opération est donc ainsi rendue très propre et à l'abri de toute contamination croisée, d'échantillon à échantillon.

De plus, l'utilisation de seringue selon l'invention évite tout danger de coupure ou de piqûre pour le manipulateur, offrant une sécurité vis-à-vis du risque biologique, ce qui n'était pas le cas dans l'art antérieur avec l'emploi de scalpel.

### Exemple 3

### Prélèvement d'échantillon végétal mou :

On a procédé à une série de prélèvements sur plusieurs bananes à l'aide d'une seringue de l'invention obtenu selon l'exemple 1.

Elle a permis de mesurer aisément et reproductiblement une masse constante de banane.

Les essais de prélèvement ont été répétés une dizaine de fois et on a obtenu des valeurs extrêmement proches les unes des autres, confirmant ici aussi la reproductibilité de l'utilisation de la seringue selon l'invention et donc du procédé selon l'invention en prélèvement, extrusion et libération d'échantillon mou végétal.

L'invention ainsi décrite et mise en oeuvre dans les exemples précédents permet donc d'obtenir un dispositif de prélèvement d'utilisation reproductible, commode et pratique, sans danger, à usage unique et jetable, permettant d'éviter toute contamination d'un échantillon mou à l'autre. La simplicité du dispositif et sa mise en oeuvre très rapide rendent le dispositif et le protocole selon l'invention aisément adaptés au prélèvement et à la distribution, par extrusion et libération, de l'échantillon mou, par exemple du type tronc cérébral, en abattoir, en laboratoire d'analyses, etc. Le fait d'avoir des dispositifs présentant des graduations et/ou des repères visibles contribue, quant à elle, à l'excellente reproductibilité des prélèvements requise. Enfin le dispositif de l'invention peut être aisément réalisé industriellement, selon des protocoles bien connus et à bas prix.

Bien entendu, la présente invention n'est pas limitée aux exemples montrés, mais elle inclut toutes les variantes des dispositifs et procédés selon l'invention telle que définie par les revendications.

## Revendications

1. Dispositif de prélèvement d'échantillon biologique mou (20), comprenant un corps cylindrique creux (10), muni de deux ouvertures (10A et 10B), une à chaque extrémité, et dans lequel, par une première extrémité (10A), est inséré un piston (12) muni d'une tige (14), ledit ensemble piston plus tige (12, 14) pouvant être déplacé vers l'avant et vers l'arrière dans ledit corps cylindrique creux (10), l'ouverture de la deuxième extrémité (10B) du corps cylindrique creux (10) présentant un bord tranchant (10B), ladite deuxième extrémité portant au moins un fil de découpe (16, 17) qui est disposé diamétralement en travers de l'ouverture de la deuxième extrémité (10B) du corps cylindrique et est orienté perpendiculairement à l'axe dudit corps et ledit dispositif présentant des moyens de repérage visibles (22) pour repérer un volume correspondant à une variation de la position du piston (12) dans le corps cylindrique creux (10).

2. Dispositif selon la revendication 1, **caractérisé en ce que** ledit corps cylindrique creux (10) est gradué (22).

3. Dispositif selon la revendication 1, **caractérisé en ce que** ledit corps cylindrique creux (10) est pourvu d'au moins deux moyens de repérage visibles, situés entre ses deux extrémités (10A et 10B), en deux positions distinctes désirées et délimitant un volume cylindrique défini.

4. Dispositif selon la revendication 1, **caractérisé en ce que** le piston (12) est muni d'une tige (14), pourvue d'au moins deux moyens de repérage visibles, situés entre ses deux extrémités, en deux positions distinctes désirées et définissant un volume cylindrique donné.

5. Dispositif selon l'une quelconque des revendications 1 à 4, **caractérisé en ce que** le bord tranchant de la deuxième extrémité (10B) du corps cylindrique creux (10) est formé par une réduction progressive de l'épaisseur de la paroi de ce corps (10).

6. Dispositif selon l'une quelconque des revendications 1 à 5, **caractérisé en ce que** les au moins deux moyens de repérage visibles définissent au moins un pas correspondant à un volume d'échantillon à prélever ou à extruder.

7. Procédé de prélèvement post-mortem d'échantillon biologique mou, à l'aide d'un dispositif selon l'une quelconque des revendications 1 à 6, comprenant les étapes suivantes :
(1) ledit dispositif est appliqué à la surface dudit échantillon, la deuxième extrémité (10B) du corps cylindrique creux (10) étant en contact direct avec ledit échantillon (20), et l'ensemble piston plus tige (12, 14) étant alors poussé à fond vers ladite deuxième extrémité (10B),
(2) ledit corps cylindrique creux est enfoncé jusqu'au niveau désiré dans ledit échantillon, alors que le piston est maintenu au niveau de la surface dudit échantillon,
(3) lorsque le niveau désiré d'enfoncement dudit corps cylindrique creux est atteint, ledit corps cylindrique creux subit une rotation sur son axe pour découper l'échantillon (20) à l'aide d'au moins un fil de découpe (16, 17),
(4) l'ensemble constitué dudit corps cylindrique creux et dudit ensemble piston plus tige, maintenu tel quel, est retiré de l'échantillon.

8. Procédé selon la revendication 7, **caractérisé en ce qu'**il comprend en plus l'étape
(5) le volume d'échantillon désiré est extrudé puis libéré dans un récipient approprié.

9. Procédé selon la revendication 7 ou la revendication 8, **caractérisé en ce que** l'échantillon prélevé (20A) est extrudé du corps cylindrique creux (10) du dispositif pour être libéré dans un récipient approprié en vue de la détection et/ou quantification de l'analyte contenu dans l'échantillon (20).

10. Procédé selon l'une quelconque des revendications 7 à 9, réalisé à l'aide d'un dispositif selon la revendication 1, **caractérisé en ce que** les moyens de repérage visibles sont utilisés pour repérer le volume d'échantillon prélevé, extrudé et/ou libéré.

11. Procédé selon la revendication 9, réalisé à l'aide d'un dispositif selon la revendication 1, **caractérisé en ce que** les moyens de repérage visibles sont utilisés pour repérer le volume d'échantillon distribué, extrudé et/ou libéré dans le récipient.

12. Procédé selon l'une quelconque des revendications 7 à 11, **caractérisé en ce que**, pour découper l'échantillon (20) à l'aide du fil de découpe (16, 17), on fait tourner le corps cylindrique creux (10) par rotation, notamment sur au moins 180 degrés.

13. Utilisation d'un dispositif selon l'une quelconque des revendications 1 à 6, pour prélever post-mortem un échantillon mou de matière biologique animale, en vue de la détection d'une molécule d'analyte ou marqueur diagnostique d'une encéphalopathie spongiforme transmissible.

14. Utilisation selon la revendication 13, pour prélever un échantillon de tronc cérébral bovin, notamment centré sur les noyaux sensitifs et moteurs du nerf vague.

## Claims

1. Device for collecting a soft biological sample (20), comprising a hollow cylindrical body (10) with two openings (10A and 10B), one at each end, wherein a piston (12) with a rod (14) is inserted via a first end (10A) and said piston-and-rod assembly (12, 14) can be displaced back and forth inside said hollow cylindrical body (10), the opening in the second end (10B) of the hollow cylindrical body (10) having a slicing edge (10B) and said second end carrying at least one cutting wire (16, 17) which is arranged diametrically across the opening in the second end (10B) of the cylindrical body and is oriented perpendicularly to the axis of said body and said device having visible identification means (22) for identifying a volume corresponding to a variation in the position of the piston (12) in the hollow cylindrical body (10).

2. Device according to claim 1, **characterized in that** said hollow cylindrical body (10) is graduated (22).

3. Device according to claim 1, **characterized in that** said hollow cylindrical body (10) is provided with at least two visible marks located between its two ends (10A and 10B), in two different desired positions delimiting a given cylindrical volume.

4. Device according to claim 1, **characterized in that** the piston (12) is fitted with a rod (14) provided with at least two visible marks located between its two ends, in two different desired positions defining a given cylindrical volume.

5. Device according to any one of claims 1 to 4, **characterized in that** the slicing edge of the second end (10B) of the hollow cylindrical body (10) is formed by a gradual reduction in the thickness of the wall of this body (10).

6. Device according to any one of claims 1 to 5, **characterized in that** the at least two visible identification marks define at least one pitch corresponding to a volume of sample to be collected or extruded.

7. Method of collecting post-mortem a soft biological sample using a device according to any one of claims 1 to 6, comprising the following steps:
(1) said device is applied to the surface of said sample, the second end (10B) of the hollow cylindrical body (10) being in direct contact with said sample (20), and the piston-and-rod assembly (12, 14) is then pushed as far as it will go towards said second end (10B),
(2) said hollow cylindrical body is pushed into said sample to the desired depth while the piston is kept at the surface of said sample,
(3) when said hollow cylindrical body has reached the desired depth of penetration, said hollow cylindrical body is rotated about its axis to cut the sample (20) by means of at least one cutting wire (16, 17), and
(4) said hollow cylindrical body and said piston-and-rod assembly, kept as such, are withdrawn from the sample together.

8. Method according to claim 7, **characterized in that** it also comprises the following step:
(5) the desired volume of sample is extruded and then released into an appropriate container.

9. Method according to claim 7 or claim 8, **characterized in that** the collected sample (20A) is extruded from the hollow cylindrical body (10) of the device and released into an appropriate container for detection and/or quantification of the analyte contained in the sample (20).

10. Method according to any one of claims 7 to 9 carried out using a device according to claim 1, **characterized in that** the visible identification means are utilized to identify the volume of sample collected, extruded and/or released.

11. Method according to claim 9 carried out using a device according to claim 1, **characterized in that** the identification means or visible marks are utilized to identify the volume of sample dispensed, extruded and/or released into the container.

12. Method according to any one of claims 7 to 11, **characterized in that**, to cut the sample (20) by means of the cutting wire (16, 17), the hollow cylindrical body (10) is rotated, especially through at least 180 degrees.

13. Use of a device according to any one of claims 1 to 6 for collecting post-mortem a soft sample of animal biological material, for detection of a molecule of analyte or diagnostic marker of a transmissible spongiform encephalopathy.

14. Use according to claim 13 for collecting a sample of bovine brain stem, especially that which is centered on the sensory and motor nuclei of the vagus nerve.

## Patentansprüche

1. Vorrichtung zur Entnahme einer weichen biologischen Probe (20), die einen zylindrischen Hohlkörper (10) aufweist, der mit zwei Öffnungen (10A und 10B), an jedem Ende eine, versehen ist, und in den über ein erstes Ende (10A) ein mit einer Stange (14) versehener Kolben (12) eingefügt ist, wobei die Einheit aus Kolben und Stange (12, 14) im zylindrischen Hohlkörper (10) nach vorne und nach hinten verschoben werden kann, wobei die Öffnung des zweiten Endes (10B) des zylindrischen Hohlkörpers (10) einen Schneiderand (10B) aufweist, wobei das zweite Ende mindestens einen Schneidedraht (16, 17) trägt, der diametral quer über die Öffnung des zweiten Endes (10B) des zylindrischen Hohlkörpers angeordnet und senkrecht zur Achse des Körpers ausgerichtet ist, und wobei die Vorrichtung sichtbare Markierungsmittel (22) aufweist, um ein Volumen zu markieren, das einer Veränderung der Stellung des Kolbens (12) im zylindrischen Hohlkörper (10) entspricht.

2. Vorrichtung nach Anspruch 1, **dadurch gekennzeichnet, dass** der zylindrische Hohlkörper (10) eine Gradeinteilung (22) aufweist.

3. Vorrichtung nach Anspruch 1, **dadurch gekennzeichnet, dass** der zylindrische Hohlkörper (10) mit mindestens zwei sichtbaren Markierungsmitteln versehen ist, die sich zwischen seinen beiden Enden (10A und 10B) an zwei unterschiedlichen gewünschten Stellen befinden und ein definiertes zylindrisches Volumen begrenzen.

4. Vorrichtung nach Anspruch 1, **dadurch gekennzeichnet, dass** der Kolben (12) mit einer Stange (14) ausgestattet ist, die mit mindestens zwei sichtbaren Markierungsmitteln versehen ist, die sich zwischen ihren beiden Enden an zwei unterschiedlichen gewünschten Stellen befinden und ein gegebenes zylindrisches Volumen definieren.

5. Vorrichtung nach einem der Ansprüche 1 bis 4, **dadurch gekennzeichnet, dass** der Schneiderand des zweiten Endes (10B) des zylindrischen Hohlkörpers (10) durch eine progressive Verringerung der Stärke der Wand dieses Körpers (10) gebildet wird.

6. Vorrichtung nach einem der Ansprüche 1 bis 5, **dadurch gekennzeichnet, dass** die mindestens zwei sichtbaren Markierungsmittel mindestens einen Schritt definieren, der einem Volumen einer zu entnehmenden oder zu extrudierenden Probe entspricht.

7. Verfahren zur Post-mortem-Entnahme einer weichen biologischen Probe mit Hilfe einer Vorrichtung gemäß einem der Ansprüche 1 bis 6, das die folgenden Schritte aufweist:
(1) die Vorrichtung wird auf die Oberfläche der Probe aufgebracht, wobei das zweite Ende (10B) des zylindrischen Hohlkörpers (10) in direktem Kontakt mit der Probe (20) steht, und die Einheit aus Kolben und Stange (12, 14) dann ganz nach vorne zum zweiten Ende (10B) hin gestoßen wird,
(2) der zylindrische Hohlkörper wird bis zur gewünschte Tiefe in die Probe eingedrückt, während der Kolben in Höhe der Oberfläche der Probe gehalten wird,
(3) wenn die gewünschte Eindrücktiefe des zylindrischen Hohlkörpers erreicht ist, wird dem zylindrischen Hohlkörper eine Drehung um seine Achse verliehen, um die Probe (20) mit Hilfe von mindestens einem Schneidedraht (16, 17) zu zerschneiden,
(4) die aus dem zylindrischen Hohlkörper und der Einheit aus Kolben und Stange bestehende Einheit wird unter Beibehaltung ihrer Stellung aus der Probe herausgezogen.

8. Verfahren nach Anspruch 7, **dadurch gekennzeichnet, dass** es außerdem den folgenden Schritt enthält
(5) das gewünschte Probenvolumen wird extrudiert und dann in einen geeigneten Behälter freigesetzt.

9. Verfahren nach Anspruch 7 oder Anspruch 8, **dadurch gekennzeichnet, dass** die entnommene Probe (20A) aus dem zylindrischen Hohlkörper (10) der Vorrichtung extrudiert wird, um in einen geeigneten Behälter zur Erfassung und/oder Quantifikation des in der Probe (20) enthaltenen Analyten freigesetzt zu werden.

10. Verfahren nach einem der Ansprüche 7 bis 9, das mit Hilfe einer Vorrichtung nach Anspruch 1 durchgeführt wird, **dadurch gekennzeichnet, dass** die sichtbaren Markierungsmittel verwendet werden, um das entnommene, extrudierte und/oder freigesetzte Probenvolumen zu markieren.

11. Verfahren nach Anspruch 9, das mit Hilfe einer Vorrichtung nach Anspruch 1 durchgeführt wird, **dadurch gekennzeichnet, dass** die sichtbaren Markierungsmittel verwendet werden, um das verteilte, extrudierte und/oder in den Behälter freigesetzte Probenvolumen zu markieren.

12. Verfahren nach einem der Ansprüche 7 bis 11, **dadurch gekennzeichnet, dass** zum Zerschneiden der Probe (20) mit Hilfe des Schneidedrahts (16, 17) dem zylindrischen Hohlkörper (10) eine Drehung von insbesondere mindestens 180 Grad verliehen wird.

13. Verwendung einer Vorrichtung nach einem der Ansprüche 1 bis 6, um eine weiche Probe einer biologischen Tiersubstanz zum Zweck der Erfassung eines Analytmoleküls oder eines Diagnosemarkers einer übertragbaren spongiformen Enzephalopathie post mortem zu entnehmen.

14. Verwendung nach Anspruch 13, um eine Probe eines Rinderstammhirns zu entnehmen, die insbesondere auf die sensitiven und motorischen Nuklei des Nervus Vagus zentriert ist.
